(19) Europäisches Patentamt
European Patent Office
Office européen des brevets

(11) Publication number: **0 456 308 A1**

# (12) EUROPEAN PATENT APPLICATION

(21) Application number: **91201043.6**

(51) Int. Cl.[5]: **G01N 33/76**, G01N 33/543

(22) Date of filing: **02.05.91**

(30) Priority: **11.05.90 US 522441**
**18.06.90 US 539780**

(43) Date of publication of application:
**13.11.91 Bulletin 91/46**

(84) Designated Contracting States:
**AT BE CH DE DK FR GB IT LI LU NL SE**

(71) Applicant: **EASTMAN KODAK COMPANY**
**343 State Street**
**Rochester, New York 14650-2201(US)**

(72) Inventor: **Warren, Harold Chester, c/o**
**Eastman Kodak Company**
**Patent Department, 343 State Street**
**Rochester, New York 14650-2201(US)**
Inventor: **Boyer, Bradley Porter, c/o Eastman**
**Kodak Company**
**Patent Department, 343 State Street**
**Rochester, New York 14650-2201(US)**
Inventor: **Snodgrass, Gary Louis, c/o Eastman**
**Kodak Company**
**Patent Department, 343 State Street**
**Rochester, New York 14650-2201(US)**

(74) Representative: **Baron, Paul Alexander**
**Clifford et al**
**Kodak Limited Patent Department Headstone**
**Drive**
**Harrow Middlesex HA1 4TY(GB)**

## (54) Immunoreagent composition, test kit and rapid assay for human chorionic gonadotropin.

(57) A rapid immunometric assay for human chorionic gonadotropin (hCG), commonly used for detection of pregnancy in human females, has been developed using a specific wash solution and conjugate level which reduces background and improves sensitivity. The assay can be carried out within two minutes using a microporous membrane to capture the "sandwich" complex of antigen and two antibodies, one of which is located on the membrane. Greater sensitivity at low levels of hCG have been demonstrated so the assay is useful as an early indicator of pregnancy. An immunoreagent composition and test kit are provided for use in the assay.

EP 0 456 308 A1

The present invention relates to a novel and rapid assay for hCG that can be used as an early pregnancy indicator for human females. It also relates to an aqueous immunoreagent composition and a diagnostic test kit useful in the assay.

There is a continuing need in the art to have a test which will rapidly and accurately detect low concentrations of human chorionic gonadotropin (hCG) as an early pregnancy indicator. This hormone increases in concentration in a human female soon after conception, and it is desirable to detect it as early as possible, even at a concentration as low as 50 mI.U./ml. Many commercial tests have been developed and marketed with the aim of providing sensitive pregnancy indication within 10 minutes, but not every test has successfully met all criteria set by doctors and other professionals which carry out such tests.

A kit useful for the determination of hCG has been marketed since early 1988 by Eastman Kodak Company as the Kodak Surecell™ hCG-Urine Test Kit. This kit contains various reagents and equipment needed for performing the assay sensitively within five minutes. Further details about this assay and kit are described, for example, in US-A-4,870,007. The assay is carried out using a disposable test device having microporous membranes in test wells which "capture" the complex of hCG with two or more antibodies, one of which is immobilized on small particles through an avidin-biotin bond. A wash solution containing sodium phosphate and sodium dodecyl sulfate (10 mmolar) was used to separate uncomplexed materials from the complex through the membrane.

It was later recognized that sodium dodecyl sulfate reduced the background in hCG assays (see EP-A-0 328 388) as long as it was present in an amount of at least 0.01 weight %. Unwanted background is observed dye or other signal generated by sources other than hCG. When background is present, it is impossible to detect pregnancy early. If it is high enough, there may be unacceptable incidences of false negatives in the testing.

Still another improvement is described in EP-A-0 328 413 wherein a lower alkyl sulfate (such as sodium decyl sulfate) in the wash solution overcomes a crystallization problem found with long-term storage of wash solutions containing sodium dodecyl sulfate. Moreover, more than six times the amount of the lower alkyl sulfate is used compared to the former wash solution.

In carrying out immunometric assays (or what are commonly known as sandwich assays) using the microporous membranes noted above, it has been advantageous to have the assay provide a detectable signal (such as a dye) in a defined region of the membrane, such as defined circle or emblem. The membrane outside of the defined region can then be used to observe background. If the assay is run properly, the background will be negligible. It has been noticed, however, that the earlier assays sometimes show a detectable "ring" of background color on the membrane outside the defined region. This background is not entirely eliminated using the improvements of earlier applications.

Moreover, there is a need to improve the sensitivity and rapidity of the earlier assays. It is desired to consistently detect as low as 50 mI.U./ml of hCG which is normally present in female urine as soon as 1 week after conception. To detect this low amount in a rapid manner, that is, within less than 2 minutes, is a highly desired object, but one which has not been readily achieved with known assays.

The problems noted above with known essays for hCG are overcome with an immunometric assay for the determination of human chorionic gonadotropin comprising:

A. contacting a specimen suspected of containing human chorionic gonadotropin with a microporous membrane having thereon a first antibody directed to one epitopic site of hCG,

to form a complex of the first antibody and hCG on the membrane,

B. without incubation (preferably immediately) contacting the complex with a solution of a second antibody directed to a different epitopic site of hCG, the second antibody being conjugated to an enzyme and said conjugate being present in an aqueous composition at a concentration of at least 2 $\mu$g/ml,

to form a sandwich complex of first and second antibodies with hCG on the membrane,

C. without incubation (preferably immediately) after step B, washing the sandwich complex at least once with a wash solution comprising a lower alcohol sulfate and a salt which is present in an amount sufficient to provide an ionic strength of from 0.1 to 1,

D. without incubation (preferably immediately) after step C, contacting the sandwich complex on the membrane with a composition which provides a dye in the presence of the enzyme, and

E. within 1 minute of step D, determining the presence of dye on the membrane as an indication of the presence or amount of hCG in the specimen.

Moreover, an immunometric assay for the determination of human chorionic gonadotropin comprises:

A. forming a sandwich complex of human chorionic gonadotropin with first and second antibodies, each of the antibodies directed to a different epitopic site, the complex being captured on a microporous membrane,

and the first antibody being conjugated to an enzyme and the conjugate being contacted with hCG in

an aqueous composition at a concentration of at least 2 µg/ml,

B. without incubation (preferably immediately) after formation of the complex, washing it at least once with a wash solution comprising a lower alcohol sulfate and a salt which is present in an amount sufficient to provide an ionic strength of from 0.1 to 1,

C. without incubation (preferably immediately) after step B, contacting the sandwich complex with a composition which provides a dye in the presence of the enzyme, and

D. within 1 minute of step C, determining the presence of dye on the membrane as an indication of the presence or amount of hCG in the specimen.

Further a diagnostic test kit for the determination of hCG comprises:

a. an aqueous composition comprising a conjugate of an antibody directed to hCG and an enzyme label, and

b. a dye-providing composition which provides a dye in the presence of the enzyme label,

the kit characterized wherein the conjugate is present in an amount of at least 2 µg/ml.

The immunometric assay of this invention is rapid and highly sensitive, that is, detecting hCG levels as low as 50 mI.U./ml, as a reliable early indicator of pregnancy. The assay can be carried out in less than 2 minutes, and usually in less than 1 minute, making it a highly desirable product for doctors' offices and clinics. Moreover, the background often observed in known assays when membranes are used as the detection substrate is substantially reduced or eliminated, particularly when the background is seen in the form of a color "ring" around a circular detection region.

These advantages are achieved in this invention by a combination of important features. One antibody of the sandwich is located on the membrane, preferably bound to small polymeric particles which are retained on the membrane. Moreover, an enzyme-labeled antibody conjugate used in the assay is present in an amount of at least 2 µg/ml as opposed to lower amounts (for example 1.125 µg/ml or less) used in known assays. The sandwich complex formed among antigen and the two antibodies is washed on the membrane using a wash solution containing a lower alcohol sulfate and a salt, the solution having an ionic strength of from 0.1 to 1. The steps of the assay are carried out immediately after one another as fluid is quickly drained through the membrane and the desired complex is captured on its surface.

The present invention is an immunometric assay for hCG which is typically found in the urine of a pregnant human female. The assay is carried out using two antibodies which are directed to different epitopic sites of the antigen so that neither antibody inhibits complexation of the other with the antigen.

The antibodies can be monoclonal or polyclonal, and can be obtained from a number of commercial sources, or prepared using known procedures. Polyclonal antibodies are generally prepared by injecting a suitable host animal with the antigen and collecting the antibodies formed in the animal's blood. Monoclonal antibodies are prepared using suitable hybridoma cells and known techniques. Preferably, the assay of this invention is carried out using one monoclonal antibody and one polyclonal antibody.

The assay is carried out by contacting a specimen (usually, but not limited to, urine) suspected of containing hCG with a microporous filtration membrane which has a porosity to retain complexed materials while allowing uncomplexed materials and fluid to drain rapidly. Such membranes can be constructed on any natural or synthetic material which retains its integrity in contact with fluids. Generally, the membranes are made of polymeric materials such as polyesters, polyamides, polethyleneimines, polycarbonates, cellulosic materials and addition polymers prepared from ethylenically unsaturated polymerizable monomers. They can be charged , but preferably they are nonionic. They can be uncoated or coated with surfactants or other materials which enhance assay performance and fluid drainage. The membrane pore size varies with the size of particles (described below) used in the assay, but generally the average pore size is from 1 to 10 µm.

Particularly useful membranes are prepared from a polyamide (a long-chain polymer having recurring amide groups in the backbone). Representative materials include, but are not limited to, polyhexamethylene dodecanediamide (nylon 612), polyhexamethylene adipamide (nylon 66), polyhexamethylene sebacamide (nylon 610), poly-ε-caprolactam (nylon 6), poly-7-aminoheptaneamide (nylon 7) and mixtures thereof. Nylon 66 is preferred. Useful microporous membranes are commercially available from Pall Corp. as Biodyne™ A, Ultipore™ and LoProdyne™ filtration membranes. Preferably, these membranes have an average pore size of 5 µm.

The membrane described herein can be used in combination with other equipment (beakers, bottles, test tubes or other containers) in order to carry out the assay. The operator can hand-hold the membrane over a container to catch the fluid. Alternatively, and preferably, the membrane is mounted in a test device, the configuration of which is immaterial since there are many known in the art, including those in US-A-3,825,410, US-A-3,888,629, US-A-3,970,429 and US-A-4,446,232. Particularly useful test devices are described in US-A-4,833,087 and US-A-4,921,677 and are commercially available as Surecell™ test devices

from Eastman Kodak Co.

Where the membrane is mounted in a test device, it is preferred that the device have more than one test well or region so that control assays can be carried out simultaneously. In the preferred Surecell™ test devices, there are three test wells, each having a membrane mounted therein, and two of the test wells being designed for control assays. Other variations of test devices would be within the skill of the ordinary worker in the art.

The remainder of the discussion will be provided in view of preferred embodiments using disposable test devices, but it should be understood that the individual steps and features of the assays could be readily adapted for the use of membranes outside of test devices.

The microporous membrane has thereon a first antibody which forms a complex with any hCG present in the specimen. The antibody can be adsorbed to the membrane or covalently bound thereto in some manner using reactive groups which are well understood in the art. For example, US-A-4,727,019 describes the binding of receptors (such as antibodies) to a membrane or filter for sandwich assays. It is important to consider, however, that the antibody used in this invention is not embedded or entrapped within the pores of the membrane, but is substantially on its outer surfaces, unlike the reagents described in EP-A-0 200 381.

Preferably, the antibody is covalently bound to polymeric particles which are composed of polymers having the requisite surface reactive groups for directly or indirectly (through a linking moiety) attaching antibody molecules. The many types of particles, reactive groups and attachment chemistry are too numerous to mention here and are well known in the art. Representative materials and attachment chemistry are described in US-A-4,808,524, US-A-4,828,978 and EP-A-0 308 235.

Thus, the polymeric particles generally have reactive groups which include, but are not limited to, carboxyl, epoxy, aldehyde, haloalkyl, activated 2-substituted ethylsulfonyl, vinylsulfonyl, vinylcarbonyl and others known in the art to react with reactive amine or sulfhydryl groups of the antibodies. Preferred reactive groups are carboxyl, haloalkyl, activated 2-substituted ethylsulfonyl and vinylsulfonyl.

Particularly useful polymeric particles are composed of a polymer represented by the structure:

$$-(A)_x-(B)_{100-x}$$

wherein A represents recurring units derived from one or more ethylenically unsaturated polymerizable monomers containing carboxyl groups or salts thereof or precursors of such groups, and B represents recurring units derived from one or more ethylenically unsaturated polymerizable monomers other than those from which A is derived, and x is from 0.1 to 70 mole percent.

Preferably, B is derived from styrene or a styrene derivative, an acrylic or methacrylic acid ester, or acrylonitrile, and x is from 1 to 20 mole percent.

In the noted structure, A can be derived from acrylic acid, methacrylic acid, itaconic acid, β-carboxyethyl acrylate, β-carboxyethyl methacrylate, *m* & *p*-carboxymethylstyrene, methacrylamidohexanoic acid or N-(2-carboxy-1,1-dimethylethyl)acrylamide, or a salt or anhydride precursor thereof, and others known in the art.

Most preferably, the monomers from which A is derived are represented by the structure:

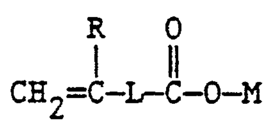

wherein R is hydrogen, halo or alkyl of 1 to 3 carbon atoms, M is hydrogen, an alkali metal ion or an ammonium ion and L is an organic linking group having from 8 to 50 atoms selected from the group consisting of carbon, nitrogen, oxygen and sulfur atoms in the linking chain.

Specific embodiments of such monomers as defined wherein R is hydrogen or methyl, M is hydrogen or an alkali metal ion, and L comprises two or more alkylene or arylenealkylene groups which are connected or terminated with an oxy, thio, imino ($-NR^1-$), carbonyloxy (-COO-), carbonylimino ($-CONR^1-$), ureylene ($-NR^1CONR^1-$) or sulfonylimino ($-SO_2NR^1-$) group, wherein each $R^1$ is independently hydrogen, alkyl having 1 to 10 carbon atoms, cycloalkyl having 4 to 10 carbon atoms or aryl having 6 to 14 carbon atoms.

Antibodies can be attached to polymeric particles having the requisite reactive groups using chemistry

well known in the art. For those particles having surface carboxy groups, attachment can be achieved using known carbodiimide chemistry, carbamoylonium compounds (such as described in EP-A-0 308 235) and other known activating agents. Antibodies are preferably attached to such polymeric particles using 1-(1-pyrrolidinylcarbonyl)pyridinium chloride as an activating agent, as described in Example 1 below.

Immunoreagents composed of antibodies attached to polymeric particles are applied to the microporous membrane in any suitable manner in order to make them available for complexation with antigen in the test specimen. Generally, they are applied in a suspension containing a small amount of a binder material (such as polyacrylamide) and dried in a distinct region of the membrane. The binder serves to keep the reagents in suspension prior to application and provides some physical stability on the membrane. However, while the reagents do not readily come off the membrane when contacted by specimen, they are not immobilized to the extent that they cannot be dislodged from the membrane when some force is applied.

Specimen applied to the membrane readily drains through the pores but antigen is complexed with the antibodies thereon. This complexation occurs immediately upon contact as well as during the few seconds it takes the operator to begin the next step of the assay. There is no incubation of the antigen with the first antibody as is commonly done in known assays.

The complex is then contacted with a conjugate formed of a second antibody to hCG and an enzyme to form a sandwich complex on the membrane. Reagents and procedures for attaching enzymes to antibodies are readily available in the literature and from commercial sources. Conjugate formation is described, for example, in US-A-4,361,647, Engvall and others, J. Immunol., 109, pp. 129-35, 1972, Imagawa and others, J.Appl.Biochem., 4, pp. 51-57, 1982, and Yoshitake and others, Eur.J.Biochem., 101, 395, 1979. Useful enzymes include, but are not limited to, peroxidase, alkaline phosphatase, malate dehydrogenase, glucose oxidase, urease, catalase, glucose-6-phosphate dehydrogenase, β-galactosidase, glucoamylase, acetylcholineesterase, lysozyme, β-amylase and acid phosphatase. Peroxidase and alkaline phosphatase are preferred with peroxidase being most preferred. The conjugate is present in an aqueous composition amount of at least 2 µg/ml, and preferably from 2.5 to 5 µg/ml. More can be used, but the advantages do not increase significantly above these amounts.

Generally, the immunoreagent composition comprising the conjugate is buffered with one or more suitable buffers (such as those noted below) at a pH of from 5 to 9. Optional components of the composition include nonimmunological proteins (that is, proteins which do not bind in immunological reaction with hCG) such as casein, α-casein, fetal bovine serum and porcine gamma globulin, and amphoteric surfactants, such as Lonzaine™ C amphoteric surfactant (Lonza Corp.) and others known in the art such as those described in EP-A-0 363 091.

The sandwich complex of antigen with the two antibodies is retained on the membrane while fluid and uncomplexed materials drain through. Immediately, the sandwich complex is washed at least once, and preferably twice, with a specific wash solution. Thus, there is no incubation of the antigen-first antibody complex with the second antibody to form the sandwich complex.

The wash solution is an aqueous buffered solution containing a lower alcohol sulfate having from 6 to 10 carbon atoms (either linear or branched chain), and a cation which is generally an alkali metal (for example, sodium, lithium and potassium) or ammonium. Representative sulfates include ammonium decyl sulfate, sodium decyl sulfate, sodium octyl sulfate, rubidium decyl sulfate, lithium hexyl sulfate and potassium heptyl sulfate. Sodium decyl sulfate is preferred, and other details of these materials are found in EP-A-0 328 413.

The sulfate is present in the wash solution in a concentration of at least 1.5, and preferably from 1.8 to 4, weight percent (based on total solution weight).

The solution is buffered to a pH of from 5 to 9, and preferably from 7 to 8, using one or more suitable buffers. Such buffers include, but are not limited to, sodium phosphate, N-(2-acetamido)-2-aminoethanesulfonic acid, N,N-bis(2-hydroxyethyl)glycine, 4-(2-hydroxyethyl)-1-piperazineethanesulfonic acid, 4-morpholine-propanesulfonic acid, 2-[tris(hydroxymethyl)methylamino]-1-ethanesulfonic acid, N-[tris-(hydroxymethyl)methyl]glycine and tris(hydroxymethyl)aminomethane. Sodium phosphate buffer is preferred.

The wash solution has an ionic strength of from 0.1 to 1 with an ionic strength of from 0.4 to 0.7 being preferred. The required ionic strength may be provided partially by the presence of ions from the buffer, but it is also provided by the presence of one or more mono- or divalent water-soluble salts. Useful salts have an alkali metal, alkaline earth metal or ammonium cation and include sodium chloride, ammonium chloride, potassium iodide, lithium chloride, sodium bromide, magnesium bromide, calcium chloride and others readily apparent to one skilled in the art. Monovalent salts (such as alkali metal salts) are preferred and sodium chloride is most preferred.

Thus, one or more of such salts are present in an amount sufficient to provide the desired ionic

strength. It is understood that if the ionic strength is provided solely by one or more salts, the concentration of salt will be higher than if the ionic strength is partially provided by anions from buffers or other components in the wash solution.

The sandwich complex on the membrane is preferably washed twice with the wash solution although additional washings are not harmful. But, in order to reduce the time of the assay as much as possible, washing once or twice is usually sufficient.

Immediately (that is, without incubation or any delay), the washed sandwich complex on the membrane is contacted with a composition which provides a dye in the presence of the enzyme. This composition generally contains a substrate for the enzyme, or one or more reagents that act with a substrate separately supplied to form a dye if enzyme is present. The dye can be formed from a chemical reaction of the substrate itself, or it can be formed from the reaction product of two or more other compounds enzymatically converted by the enzyme. Appropriate substrates would be readily apparent to one skilled in the art for each useful enzyme.

In a particularly preferred embodiment, the enzyme label is peroxidase, and hydrogen peroxide and a suitable dye-providing composition are added to provide a dye signal on the membrane where peroxidase is present in a sandwich complex. Reagents useful with peroxidase include triarylimidazole leuco dyes (as described in US-A-4,089,747), other imidazole compounds and benzidine and benzidine derivatives.

Addition of the dye-providing composition takes but a few seconds, and almost immediately a dye is seen on the membrane if the sandwich complex is present. Generally, the dye signal is evaluated visually or with spectrophotometric equipment within a few seconds and up to 1 minute in order to evaluate the presence or amount of hCG in the test specimen. Negative and positive controls (where present) can also be evaluated at the same time, or within a few seconds later. It should be understood that in some cases, an operator might not observe the dye signal right away because he or she may carry out other duties during that time. The point remains, however, that the dye signal is observable within a very short time even if this improvement is not always taken advantage of.

In a preferred embodiment, an immunometric assay for the determination of human chorionic gonadotropin comprises:

A. contacting a urine sample suspected of containing human chorionic gonadotropin with a microporous membrane having coated thereon a first antibody directed to one epitopic site of hCG,

the first antibody being covalently bound to polymeric particles which are composed of a polymer represented by the structure:

$$-(\ A\ )_x-(\ B\ )_{100-x}$$

wherein A represents recurring units derived from one or more ethylenically unsaturated polymerizable monomers containing carboxyl groups or salts thereof or precursors of said groups, and B represents recurring units derived from one or more ethylenically unsaturated polymerizable monomers other than those from which A is derived, and x is from 0.1 to 70 mole percent,

to form a water-insoluble complex of the first antibody and hCG on the membrane,

B. without incubation (preferably immediately) contacting the complex with an aqueous composition of a second antibody directed to a different epitopic site of hCG, the second antibody being conjugated to an enzyme and the conjugate being present in the composition at a concentration of from 2.5 to 5 $\mu$g/ml,

to form a water-insoluble sandwich complex of first and second antibodies with hCG on the membrane,

C. without incubation (preferably immediately) after step B, washing the sandwich complex at least once with a wash solution comprising sodium decyl sulfate and a monovalent salt present in an amount sufficient to provide an ionic strength of from 0.4 to 0.7,

D. without incubation (preferably immediately) after step C, contacting the sandwich complex on the membrane with a composition which provides a dye in the presence of the enzyme, and E. within 1 minute of step D, determining the presence of dye on the membrane as an indication of the presence or amount of hCG in the urine sample.

The reagents, solutions and membrane used in the practice of this invention can be supplied individually or as components of a diagnostic test kit.

More particularly, a useful diagnostic test kit for the determination of hCG comprises:

a. an aqueous composition comprising a conjugate of an antibody directed to hCG and an enzyme label,

the conjugate being present in an amount of at least 2 µg/ml, and

b. a dye-providing composition which provides a dye in the presence of the enzyme label.

Optional components of the test kit include the wash solution described herein, test device containing the membrane described herein (also containing a suitable immunoreagent for hCG), pipettes, filters, instructions, and other suitable reagents or equipment useful for an assay.

The following examples are for illustrative purposes only, and not to limit the scope of the invention. All percentages are by weight, unless otherwise specified.

Example 1: Assay for Human Chorionic Gonadotropin

This example demonstrates the practice of the present invention to detect hCG in a rapid assay.

Materials:

Surecell™ disposable test devices were used containing LoProdyne™ microporous membranes (5 µm, Pall Corp.), each coated with Fluorad™ FC 135 nonionic surfactant (3M, 0.05 g/m$^2$).

An immunoreagent was prepared by covalently attaching affinity purified goat anti-hCG alpha polyclonal antibodies (OEM Concepts, Toms River, N.J.) to particles of poly[styrene-co-3-(p-vinylbenzylthio)propionic acid] (97.6:2.4 molar ratio) using 1-(1-pyrrolidinylcarbonyl)pyridinium chloride as an activating agent. The procedure for attaching the antibodies was as follows:

A suspension of polymeric particles (3% solids) was mixed with the activating agent (0.1 molar) in 2-(4-morpholino)ethanesulfonic acid buffer in a microfuge tube. The tube was capped, and rotated end-over-end at room temperature for 10 minutes. A solution (810 µl) of the polyclonal antibodies (13.2 mg/ml) was added to the tube followed by rotation end-over-end for 18 hours at room temperature. The resulting immunoreagent had about 99% of theoretical antibody covalently bound to the polymeric particles.

A composition (2 µl) comprising the immunoreagent (0.9%), polyacrylamide (5%), Uvitex™ dye (0.01%) and thimerosal preservative (0.01%) in glycine buffer (0.1 molar, pH 8.5) was deposited on a finite area of the membrane in one of the test wells (designated the sample well).

Goat gamma globulin was covalently bound to the same type of particles using the same procedure and the resulting immunoreagent was deposited onto the membrane in another test well (designated the negative control well). A third test well (designated the positive control well) contained anti-hCG antibodies covalently bound to the same type of particles beads and hCG antigen prebound to the antibodies.

The test solution contained hCG (50 mI.U./ml) in a solution of phosphate buffered saline solution (150 mmolar sodium chloride, 50 mmolar sodium phosphate, pH 6.2), bovine serum albumin (0.7%) and merthiolate (0.01%).

A conjugate of anti-hCG monoclonal antibodies (Cambridge Medical Diagnostics) and horseradish peroxidase (Miles) was prepared using the procedures described by Yoshitake and others, Eur.J.Biochem., 101, 395 (1979). This conjugate was mixed with Medix Peroxidase Diluent (Medix Biotech, Inc. Foster City, California) containing Lonzaine™ C amphoteric surfactant (0.1%, Lonza Corp.). The final conjugate concentration in this aqueous composition was 3.38 µg/ml.

A wash solution was prepared from sodium phosphate (0.1 molar, pH 7.2), sodium decyl sulfate (100 mmolar, 2.7%), sodium chloride (0.3 molar) and thimerosal (0.01%). The ionic strength of the wash solution was about 0.6.

A dye-providing composition comprised 2-(4-hydroxy-3-methoxyphenyl)-4,5-bis(4-methoxyphenyl)-imidazole leuco dye (0.005%), poly(vinylpyrrolidone) (1%), sodium phosphate buffer (5 mmolar, pH 6.8) diethylenetriaminepentaacetic acid (10 µmolar), 4'-hydroxyacetanilide (2 mmolar) and hydrogen peroxide (10 mmolar).

Assay Procedure:

The test solution (150 µl) containing hCG (50 mI.U./ml) was added to the three test wells of the test device, and the fluids were allowed to drain through the membranes. The buffered composition containing labeled antibody (1 drop, about 40 µl) was added to each test well, and allowed to drain through. The test wells were washed twice (each time with about 300 µl) and allowed to drain through. The dye-providing composition (50 µl) was then added to each test well and allowed to drain through. After a brief incubation at room temperature, the dye density on the membranes was evaluated and scored against a color chart for dye density with 10 representing the highest density and zero the lowest density. The areas around the applied compositions in the test wells were evaluated as background. The assay was carried out three

times.

The results are provided in the following Table I as visual dye densities seen in the specific test wells for each of the three tests:

TABLE I

| Negative Control Well | | Sample Well | | Positive Control Well | |
|---|---|---|---|---|---|
| Test | Background | Test | Background | Test | Background |
| 0 | 0 | 3-4 | 0 | 7 | 0 |
| 0 | 0 | 4 | 0 | 7 | 0 |
| 0 | 0 | 4 | 0 | 7 | 0 |

These data show that in three separate assays, a very low concentration of hCG (50 mI.U.) can be readily detected with zero background using the assay of this invention. Moreover, the assay was carried out in less than 2 minutes.

Example 2: Assay for hCG Using Different Reagent

This example illustrates the practice of this invention using immunoreagents having polyclonal antibodies attached to different polymeric particles than described in Example 1.

Materials:

Surecell™ disposable test devices were used containing LoProdyne™ microporous membranes (5 µm, Pall Corp.), each coated with Fluorad™ FC 135 nonionic surfactant (3M, 0.05 g/m²).

The polymeric particles used were composed of poly[styrene-co-m & p-(2-chloroethylsulfonylmethyl)-styrene] (95.5:4.5 molar ratio), prepared using known starting materials and emulsion polymerization procedures, as described in EP-A-0 323 692.

A reagent was prepared by covalently attaching affinity purified goat anti-hCG alpha polyclonal antibodies (OEM Concepts, Toms River, N.J.) to the particles noted above according to the procedures described in EP-A-0 323 692: a suspension of the particles (3% solids) was mixed with borate buffer (0.1 molar, pH 8.5) in a microfuge tube. A solution (810 µl) of the polyclonal antibodies (13.2 mg/ml) was added to the tube followed by rotation end-over-end for 18 hours at room temperature. The resulting immunoreagent had about 99% of theoretical antibody covalently bound to the polymeric particles.

A composition (2 µl) comprising the immunoreagent (0.9%), polyacrylamide (5%), Uvitex™ dye (0.01%) and merthiolate preservative (0.01%) in glycine buffer (0.1 molar, pH 8.5) was deposited on a finite area (a center spot) of the membrane in one of the test wells (designated the sample well).

The test solution contained hCG (50 mI.U./ml) in a solution of phosphate buffered saline solution (150 mmolar sodium chloride, 50 mmolar sodium phosphate, pH 6.2), bovine serum albumin (0.7%) and merthiolate (0.01%).

A conjugate of anti-hCG monoclonal antibodies (Cambridge Medical Diagnostics) and horseradish peroxidase (Miles) was prepared using the procedures described by Yoshitake and others, Eur.J.Biochem., 101, 395 (1979). This conjugate was mixed with Medix Peroxidase Diluent (Medix Biotech, Inc. Foster City, California) containing Lonzaine™ C amphoteric surfactant (0.1%, Lonza Corp.). The final conjugate concentration of this aqueous composition was about 3.38 µg/ml.

A wash solution was prepared from sodium phosphate (0.1 molar, pH 7.2), sodium decyl sulfate (100 mmolar, 2.7%), sodium chloride (0.3 molar) and thimerosal (0.01%). The ionic strength of the wash solution was about 0.6.

A dye-providing composition comprised 2-(4-hydroxy-3-methoxyphenyl)-4,5-bis(4-methoxyphenyl)-imidazole leuco dye (0.005%), poly(vinyl pyrrolidone) (1%), sodium phosphate buffer (5 mmolar, pH 6.8) diethylenetriaminepentaacetic acid (10 µmolar), 4'-hydroxyacetanilide (2 mmolar) and hydrogen peroxide (10 mmolar).

Assay:

The test solutions (150 µl) each containing 50 mI.U./ml of hCG were added to the test wells of the Surecell™ test devices containing the noted particulate reagents, and the fluid was allowed to drain through the membrane. The peroxidase-labeled antibody composition (about 40 µl) was immediately added and allowed to drain. The resulting sandwich complex on the membrane was immediately washed twice with the wash solution (300 µl each time) with drainage allowed, removing uncomplexed materials from the membrane. Immediately, the dye-providing composition (50 µl) was added and allowed to drain through the membrane. After one minute, the dye formed on the membrane was evaluated and scored against a color chart numbering 0 to 10 with 10 representing the highest dye density. The area around the dye on the membrane was evaluated for background density.

The results of eight separate assays showed acceptable dye formation (average 1.9 signal as determined in Example 1) and negligible background in the determination of a low (50 mI.U./ml) level of hCG. In addition, the assays were completed in less than 2 minutes.

Example 3: Sandwich Assays Comparing Conjugate Levels

This example compares the present invention using 3.375 µg/ml of enzyme-labeled antibodies compared to a known assay using 1.125 µg/ml of the same conjugate. It shows that the sensitivity of the present assay was considerably greater than for the known assay, which is described in Example 1 of EP-A-0 328 413.

Materials:

The polymeric particles used were composed of poly[styrene-co-3-(p-vinylbenzylthio)propionic acid] (97.6:2.4 molar ratio) prepared using starting materials and emulsion polymerization procedures described above.

Surecell™ disposable test devices were used containing LoProdyne™ microporous membranes (5 µm, Pall Corp.), each coated with Fluorad™ FC 135 nonionic surfactant (3M, 0.05 $g/m^2$).

An immunoreagent was prepared by covalently attaching affinity purified goat anti-hCG alpha polyclonal antibodies (OEM Concepts, Toms River, N.J.) to the particles noted above using 1-(1-pyrrolidinylcarbonyl)-pyridinium chloride as an activating agent. The procedure for attaching the antibodies was as follows:

A suspension of polymeric particles (3% solids) was mixed with the activating agent (0.1 molar) in 2-(4-morpholino)ethanesulfonic acid buffer in a microfuge tube. The tube was capped and rotated end-over-end at room temperature for 10 minutes. A solution (810 µl) of the polyclonal antibodies (13.2 mg/ml) was added to the tube followed by rotation end-over-end for 18 hours at room temperature. The resulting immunoreagent had about 99% of theoretical antibody covalently bound to the polymeric particles.

A composition (2 µl) comprising the immunoreagent (0.9%), polyacrylamide (5%), Uvitex™ dye (0.01%) and merthiolate preservative (0.01%) in glycine buffer (0.1 molar, pH 8.5) was deposited on a finite area (a center spot) of the membrane in one of the test wells (designated the sample well).

Goat gamma globulin was covalently bound to the same type of particles using the same procedure and the resulting immunoreagent was deposited onto the membrane in another test well (designated the negative control well). A third test well (designated the positive control well) contained anti-hCG antibodies covalently bound to the same type of particles and hCG antigen prebound to the antibodies.

The test solution contained hCG (50 mI.U./ml) in a solution of phosphate buffered saline solution (150 mmolar sodium chloride, 50 mmolar sodium phosphate, pH 6.2), bovine serum albumin (0.7%) and merthiolate (0.01%).

A conjugate of anti-hCG monoclonal antibodies (Cambridge Medical Diagnostics) and horseradish peroxidase (Miles) was prepared using the procedures described by Yoshitake and others, Eur.J.Biochem., 101, 395 (1979). This conjugate was mixed with Medix Peroxidase Diluent (Medix Biotech, Inc. Foster City, California) containing Lonzaine™ C amphoteric surfactant (0.1%, Lonza Corp.). The final conjugate concentration was 3.375 µg/ml.

A Control composition was similarly prepared containing 1.125 µg/ml of the enzyme-labeled conjugate.

A wash solution was prepared from sodium phosphate (0.1 molar, pH 7.2), sodium decyl sulfate (100 mmolar, 2.7%), sodium chloride (0.3 molar) and thimerosal (0.01%). The ionic strength of the wash solution was about 0.6.

A dye-providing composition comprised 2-(4-hydroxy-3-methoxyphenyl)-4,5-bis(4-methoxyphenyl)-imidazole leuco dye (0.005%), poly(vinyl pyrrolidone) (1%), sodium phosphate buffer (5 mmolar, pH 6.8)

diethylenetriaminepentaacetic acid (10 µmolar), 4'-hydroxyacetanilide (2 mmolar) and hydrogen peroxide (10 mmolar).

Assay:

The test solutions (150 µl) each containing 50 mI.U./ml of hCG were added to the test wells of the Surecell™ test devices containing the noted immunoreagents, and the fluid was allowed to drain through the membrane. The peroxidase-labeled antibody composition (about 40 µl) was immediately added to one set of test devices and and allowed to drain. The Control conjugate composition was similarly added to another set of test devices. The resulting sandwich complex on the membrane in each device was immediately washed twice with the wash solution (300 µl each time) with drainage allowed, removing uncomplexed materials from the membrane. Immediately, the dye-providing composition (50 µl) was added and allowed to drain through the membrane. After one minute, the dye formed on the membrane in each device was evaluated and scored against a color chart numbering 0 to 10 with 10 representing the highest dye density. The area around the dye on the membrane was evaluated for background density.

The results (dye density readings) are shown in Table II below for both the Invention and Control (using Control conjugate composition). Considerably higher dye signals are obtained with the present invention indicating significantly greater sensitivity with the present invention over the Control assay. Moreover, the assay of this invention provided this sensitivity within about 2 minutes assay time.

Table II

| Control Conjugate | | Invention Conjugate | |
|---|---|---|---|
| Test | Background | Test | Background |
| 1-2 | 0 | 4 | 0 |
| 2 | 0 | 4 | 0 |
| 1-2 | 0 | 4 | 0 |
| 1-2 | 0 | 4 | 0 |

Example 4: Comparison of Wash Solutions in hCG Assays

This example illustrates the importance of using a wash solution comprising a salt in the hCG assay.

Two assays were carried out using materials and procedures described in Example 1 except that a Control wash solution was used in a Control assay, and the resulting dye signals were evaluated after 5 minutes of incubation instead of merely 1 minute. The Control wash solution did not contain sodium chloride.

The results indicated that the Control assay showed unacceptable background signal in the test wells of the test device. The assay carried out with the salt in the wash solution showed negligible background in both the test sample and negative control wells.

The dye signals were evaluated at the longer time of 5 minutes in order to better demonstrate the improvement in background shown with the wash solution containing the salt. Much quicker dye evaluation times, such as within 1 minute or less, are usually done in commercial settings because the sensitivity of the assay is very good during that time period. However, many operators testing for pregnancy in a doctor's office or clinic will carry out a number of tasks simultaneously, and a given hCG assay might be prolonged because of them. Thus, the operator may not evaluate the dye signal right away as would be done in the ideal situation.

**Claims**

**1.** An immunometric assay for the determination of human chorionic gonadotropin comprising:

A. contacting a specimen suspected of containing human chorionic gonadotropin with a microporous membrane having thereon a first antibody directed to one epitopic site of hCG,

to form a complex of the first antibody and hCG on the membrane,

B. without incubation contacting the complex with an aqueous composition of a second antibody directed to a different epitopic site of hCG, the second antibody being conjugated to an enzyme and the conjugate being present in the composition at a concentration of at least 2 $\mu$g/ml,

to form a sandwich complex of first and second antibodies with hCG on the membrane,

C. without incubation after step B, washing the sandwich complex at least once with a wash solution comprising a lower alcohol sulfate and a salt which is present in an amount sufficient to provide an ionic strength of from 0.1 to 1,

D. without incubation after step C, contacting the sandwich complex on the membrane with a composition which provides a dye in the presence of the enzyme, and

E. within 1 minute of step D, determining the presence of dye on the membrane as an indication of the presence or amount of hCG in the specimen.

**2.** The method as claimed in Claim 1 wherein the dye is determined within 2 minutes of beginning the assay.

**3.** An immunometric assay for the determination of human chorionic gonadotropin comprising:

A. forming a sandwich complex of human chorionic gonadotropin with first and second antibodies, each of the antibodies directed to a different epitopic site, the complex being captured on a microporous membrane,

and the first antibody being conjugated to an enzyme and the conjugate being contacted with hCG in an aqueous composition at a concentration of at least 2 $\mu$g/ml,

B. without incubation after formation of the complex, washing it at least once with a wash solution comprising a lower alcohol sulfate and a salt present in an amount sufficient to provide an ionic strength of from 0.1 to 1,

C. without incubation after step B, contacting the sandwich complex with a composition which provides a dye in the presence of the enzyme, and

D. within 1 minute of step C, determining the presence of dye on the membrane as an indication of the presence or amount of hCG in the specimen.

**4.** The invention as claimed in any of claims 1 to 3 wherein the salt is a monovalent salt.

**5.** The invention as claimed in any of claims 1 to 4 wherein the salt is present in the wash solution in an amount sufficient to provide an ionic strength of from 0.4 to 0.7.

**6.** The invention as claimed in any of claims 1 to 5 wherein the conjugate is present in an amount of from 2.5 to 5.0 $\mu$g/ml.

**7.** The invention as claimed in any of claims 1 to 6 wherein the first antibody is covalently bound to polymeric particles which are coated on the membrane.

**8.** The invention as claimed in Claim 7 wherein the polymeric particles are composed of a polymer represented by the structure:

$$-(\ A\ )_x-(\ B\ )_{100-x}$$

wherein A represents recurring units derived from one or more ethylenically unsaturated polymerizable monomers containing carboxyl groups or salts thereof or precursors of the groups, and B represents recurring units derived from one or more ethylenically unsaturated polymerizable monomers other than those from which A is derived, and x is from 0.1 to 70 mole percent.

**9.** The invention as claimed in claim 8 wherein A is derived from an ethylenically unsaturated polymerizable monomer represented by the structure:

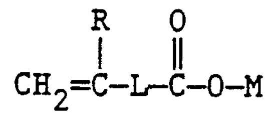

wherein R is hydrogen, halo or alkyl of 1 to 3 carbon atoms, M is hydrogen, an alkali metal ion or an ammonium ion and L is an organic linking group having from 8 to 50 atoms selected from the group consisting of carbon, nitrogen, oxygen and sulfur atoms in the linking chain.

**10.** An immunometric assay for the determination of human chorionic gonadotropin comprising:

A. contacting a urine sample suspected of containing human chorionic gonadotropin with a microporous membrane having coated thereon a first antibody directed to one epitopic site of hCG,

the first antibody being covalently bound to polymeric particles which are composed of a polymer represented by the structure:

$$-(A)_x-(B)_{100-x}$$

wherein A represents recurring units derived from one or more ethylenically unsaturated polymerizable monomers containing carboxylic acid groups or salts thereof or precursors of the groups, and B represents recurring units derived from one or more ethylenically unsaturated polymerizable monomers other than those from which A is derived, and x is from 0.1 to 70 mole percent,

to form a water-insoluble complex of the first antibody and hCG on the membrane,

B. without incubation contacting the complex with an aqueous composition of a second antibody directed to a different epitopic site of hCG, the second antibody being conjugated to an enzyme and the conjugate being present in the composition at a concentration of from 2.5 to 5 $\mu$g/ml,

to form a water-insoluble sandwich complex of first and second antibodies with hCG on the membrane,

C. without incubation after step B, washing the sandwich complex at least once with a wash solution comprising sodium decyl sulfate and a monovalent salt present in an amount sufficient to provide an ionic strength of from 0.4 to 0.7,

D. without incubation after step C, contacting the sandwich complex on the membrane with a composition which provides a dye in the presence of the enzyme, and

E. within 1 minute of step D, determining the presence of dye on the membrane as an indication of the presence or amount of hCG in the urine sample.

**11.** A diagnostic test kit for the determination of hCG comprising:

a. an aqueous composition comprising a conjugate of an antibody directed to hCG and an enzyme label, and

b. a dye-providing composition which provides a dye in the presence of the enzyme label,

the kit-characterized wherein the conjugate is present in an amount of at least 2 $\mu$g/ml.

**12.** The kit as claimed in claim 11 wherein the enzyme is selected from the group consisting of peroxidase, alkaline phosphatase, malate dehydrogenase, glucose oxidase, urease, catalase, glucose-6-phosphate dehydrogenase, $\beta$-galactosidase, glucoamylase, acetylcholineesterase, lysozyme, $\beta$-amylase and acid phosphatase.

**13.** The kit as claimed in either of claims 11 and 12 further comprising a wash solution comprising a lower alcohol sulfate and a salt which is present in an amount sufficient to provide an ionic strength of from 0.1 to 1.

**14.** The invention as claimed in any of claims 1 to 13 wherein the enzyme is peroxidase, and the dye-providing composition provides a dye in the presence of peroxidase and hydrogen peroxide.

European Patent Office

# EUROPEAN SEARCH REPORT

Application Number

**EP 91 20 1043**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 328 413 (EASTMAN KODAK CO.)<br>* Whole document * | 1-14 | G 01 N 33/76<br>G 01 N 33/543 |
| Y | WO-A-8 703 690 (MUREX CORP.)<br>* Page 8, line 1 - page 12, line 1; page 27, line 1 - page 28, line 17 * | 1-14 | |
| A,D | EP-A-0 328 388 (EASTMAN KODAK CO.)<br>* Whole document * | 1-14 | |
| | | | **TECHNICAL FIELDS SEARCHED (Int. Cl.5)** |
| | | | G 01 N |

The present search report has been drawn up for all claims

| Place of search | Date of completion of search | Examiner |
|---|---|---|
| The Hague | 19 August 91 | VAN BOHEMEN C.G. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document